(19) Europäisches Patentamt
European Patent Office
Office européen des brevets

(11) Publication number: **0 189 679 B1**

(12) **EUROPEAN PATENT SPECIFICATION**

(45) Date of publication of patent specification: **13.06.90**

(51) Int. Cl.[5]: **C 07 D 295/02,** A 61 K 31/495

(21) Application number: **85309531.3**

(22) Date of filing: **30.12.85**

(54) **Novel crystalline form of 1-benzhydryl-4-allylpiperazine dihydrochloride and method for the production thereof.**

(30) Priority: **28.12.84 BG 68185/84**

(43) Date of publication of application:
**06.08.86 Bulletin 86/32**

(45) Publication of the grant of the patent:
**13.06.90 Bulletin 90/24**

(84) Designated Contracting States:
**AT BE CH DE FR GB IT LI SE**

(56) References cited:
**EP-A-0 097 340**

**CHEMICAL ABSTRACTS, vol. 78, no. 23, 11th June 1973, page 379, column 1, abstract no. 147908w, Columbus, Ohio, US; SV. ZIKOLOVA et al.: "Analogs of N1-benzhydryl-N4-cinnamylpiperazine (cinnarizine). II. N1-Substituted-N4-benzhydrylpiperazines", & TR. NAUCHNOIZSLED. KHIM.-FARM. INST. 1972, 8, 59-67 (Cat. D) in connection with CHEMICAL SUBSTANCE INDEX, vol. 78, 1973, page 3058CS, Piperazine, 1-(diphenylmethyl)-4-(2-propenyl)-dihydrochloride**

(73) Proprietor: **SO "PHARMACHIM"**
**16, Iliensko Chaussee**
**Sofia (BG)**

(72) Inventor: **Ninov, Kiril Assenov**
**Sultan-Tepe-Street 18**
**Sofia (BG)**
Inventor: **Karakolev, Pavel Vassilev**
**Iv. Bigor St., B1.29-A**
**Sofia (BG)**
Inventor: **Mitzev, Georgi Kirilov**
**Dedeagach St. No. 27, B1.12**
**Sofia (BG)**
Inventor: **Evstatieva, Anka Veltsheva**
**Klinzi Str. B1.27-A**
**Sofia (BG)**
Inventor: **Zikolova, Tatyana Styanova**
**P. Yanev No. 31-A**
**Sofia (BG)**
Inventor: **Vitkova, Snejana Georgieva**
**N. Kamenov Str. B1.252-4**
**Sofia (BG)**
Inventor: **Nikolov, Rumen Kostov**
**Rozova Dolina St. No. 12-2**
**Sofia (BG)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 189 679 B1

Courier Press, Leamington Spa, England.

(56) References cited:

CHEMICAL ABSTRACTS, vol. 81, no. 17, 28th October 1974, page 24, column 2, abstract no. 99327m, Columbus, Ohio, US; P. MANOLOV et al.: "Phar mocological study of N'-benzhydryl-N''-allylpiperazine dihydrochloride", & FARMATSIYA (SOFIA) 1974, 24(1), 8-11

(74) Representative: Silverman, Warren et al
HASELTINE LAKE & CO. Hazlitt House 28 Southampton Buildings Chancery Lane
London WC2A 1AT (GB)

**Description**

This invention relates to a novel crystalline form of 1-benzhydryl-4-allyl-piperazine dihydrochloride and to a method for the production thereof.

Bulgarian Inventors Certificate No. 17385 discloses a group of substances which are derivatives of benzhydryl piperazine and which possess valuable pharmacological properties. One of the compounds mentioned in this patent document and which has been confirmed in a series of pharmacological studies to possess particularly valuable therapeutic properties is the compound 1-benzhydryl-4-allyl-piperazine dihydrochloride, also designated as $As_2$ and by its generic denomination aligerone:

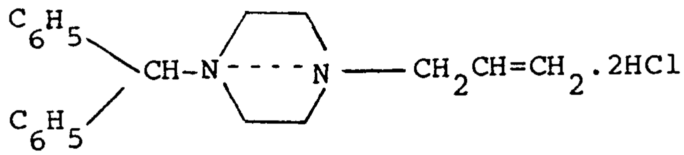

(I)

Aligerone is described by T. S. Zikolova and K. A. Ninov in an article entitled "Analogues of N-benzhydryl-N-cinnamylpiperazine"/"N-substituted-N-benzhydryl-piperazines", collection of papers, NIHFI, vol. VIII, p. 61, 1972, and also in "Drugs of the Future", 1980, 5(1) as enabling favourable circulation of blood in the brain to be achieved. It dilates the blood vessels of the brain, decreases the resistance of the blood vessels of the brain to passage of blood and increases the general rheotonalic and local blood circulation of the brain as well as the oxygen concentration in the brain tissue so that its effect is stronger than that of cinnarizine. In addition, aligerone improves the coronary and peripheric blood circulation, has a spasmolytic effect on the smoother muscle of the stomach-intestinal system and a well expressed antihistaminic effect. Finally it has been described in "Drugs of the Future", 1980, 5(1) as acting as as a nonspecific prostaglandine antagonist.

Further studies aimed at creating conditions for the wide ranging pharmacological, preclinical and clinical testing has established that 1-benzhydryl-4-allyl-piperazine dihydrochloride prepared according to methods described by Zikolova and Ninov and which will generally now be referred to hereinafter as aligerone, unless otherwise indicated, can only be prepared in a form suitable for making up in stable liquid pharmaceutical compositions with considerable difficulty.

It is an object of the present invention to provide an alternative form of aligerone having improved solubility in pharmacologically acceptable solvents and improved stability when in solution over the forms of aligerone hitherto available.

According to one aspect of the invention, there is provided an anhydrous crystalline form of 1-benzhydryl-4-allyl-piperazine dihydrochloride, hereinafter termed "crystal form "A" ", wherein it possesses absorption bands in its IR-spectra (tablets with nujol mulls) at 646 and 665 $cm^{-1}$, a ratio $A_{646}/A_{665}=0.6$, a specific triplet at 915, 926 and 952 $cm^{-1}$, a ratio $A_{926}/A_{952}=0.8$, absence of a band at 3380 $cm^{-1}$ and, a strongest diffraction X-ray band at 27.4 (2θ), corresponding to 37.8 nm.

This invention also provides a method for the preparation of this anhydrous crystalline form of aligerone dihydrochloride, which comprises providing in solution in an aqueous alcoholic medium either

(a) 1-benzhydryl-4-allylpiperazine, as such and then acidifying this solution with concentrated hydrochloric acid forming therein a crystalline precipitate of aligerone dihydrochloride and separating off the crystalline precipitate from the liquid in which it has been formed, or

(b) 1-benzhydryl-4-allylpiperazine dihydrochloride in a crystalline form other than its anhydrous crystalline form, forming therein a crystalline precipitate of aligerone dihydrochloride and then separating off the crystalline precipitate from the liquor in which it has been formed.

When 1-benzhydryl-4-allylpiperazine as such being used, the aqueous alcoholic medium provided is preferably the medium in which it has been produced, i.e. in situ operation is involved.

As a result of detailed investigations, it has been established that aligerone can exist in three crystalline forms, two polymorphous forms which may be identified as forms "A" and "B" and one pseudopolymorphous form, which is a crystalline hydrate of form "B".

The aforementioned crystalline hydrate and also polymorphous form "B" are prepared by the aforementioned method of Zikolova and Ninov by condensation of benzhydropiperazine and an allyl halide in a non-aqueous non-polar solvent, removal of the solvent and treatment of the residue with hydrochloric acid. After recrystallising from water and drying, aligerone is thus obtained by drying in a drying chamber at 80°C and its water of crystallisation is relatively easily removed if it is the anhydrous polymorphous form "B" which is to be produced.

It is polymorphous form "A" which is the novel crystalline form of the present invention. This can be prepared easily and in a reproducible manner in an aqueous-alcohol medium, from the product of condensing 1-benzhydrylpiperazine with an allylhalide in the presence of an alkaline condensation agent at a temperature of from 0 to 40°C, preferably about 25°C. Acidification of the aqueous alcohol solution of the reaction product obtained with hydrochloric acid, generally at ambient temperature, will yield polymorphous form "A". The preferred alcohols to be employed here are methanol, ethanol and propanol

whose water contents may be varied widely. A preferred reaction medium is ethanol containing from 5 to 30% by volume water.

The three crystalline forms of aligerone, namely A, B and the crystalline hydrate have been characterised by IR-spectra, X-ray diffractograms, differential thermal analysis (calorimetry) and differential gravitometry. The crystalline hydrate is identifiable as a dihydrate by following the method of K. Fischer when it is found to have a water content of 9.5% (the theoretical water content calculated for 2 molecules of water of crystallisation is 8.98%).

The various crystalline forms of aligerone are characterised by the contents of accompanying drawings wherein:

Figure 1 is an infrared spectrum of crystalline form "A";

Figure 2 is an infrared spectrum of crystalline form "B";

Figure 3 is an infrared spectrum of the crystalline dihydrate;

Figure 4 is an X-ray diffractogram of all three crystalline forms in pulverous form; and

Figure 5 shows profiles of the solubility in water of pulverous samples of the three crystalline forms.

The IR-spectra were recorded by means of a Perkin-Elmer infrared spectrometer using Nujol tablets as mulls. The spectra are disposed near the absorption bands according to frequency and this applies in particular to the two anhydrous forms "A" and "B". This makes it necessary for the purpose of characterising these forms to use the ratio between the intensities of the absorption bands which ratio differs between the different forms. In Table 1 which follows, there are presented data of infrared spectra which can be used for identifying each of the crystal modifications.

Complete characterising of the crystalline forms may be carried out utilising X-ray diffractograms of powder specimens utilising a "DRON—3" diffractometer from the Carl Zeiss Company, GDR. This diffractometer is provided with Co-K radiation and a scintillation counter. The intensity of the beam as a function of the angle 2θ is recorded at a uniform rate of increase of 1° per minute. Table 2 indicates the interplanar distances in nanometers and the relative intensities calculated for all diffraction maxima of the three modifications. Table 2 also shows the basic diffraction bands that are characteristic of the different crystalline forms.

TABLE 1

| Form "A" Cm$^{1}$ | Absorption ratio | Form "B" Cm$^{-1}$ | Absorption ratio | Cm$^{-1}$ | Dihydrate Absorption ratio |
|---|---|---|---|---|---|
| 646 | | 646 | | | |
| 665 | $A_{646}/A_{665} = 0.6$ | 665 | $A_{646}/A_{665} = 1.7$ | 770 | |
| | | | | 920 | specific band |
| 915 | characteristic | 926 | | | |
| 926 | triplet | 936 | $A_{926}/A_{952} = 1.3$ | | |
| 952 | $A_{926}/A_{952} = 0.8$ | 952 | | | |
| | | 960 | | 968 | |
| | | 1127 | specific band | 1082 | |
| | | | | 1115 | specific band |
| 1500 | | 1492 | | 1492 | |
| 3041 | characteristic | 3000 | group of bands | 3380 | very specific broad, strong band |
| 3054 | doublet | 3100 | | | |

TABLE 2

| Crystal form | (2θ) | nm | I/Io (%) |
|---|---|---|---|
| A | 25.7 not separated doublet | 40.2 | 70 |
| | 25.9 | 39.9 | 83 |
| | 27.4 | 37.8 | 100 |
| | 37.0 | 28.2 | 84 |
| B | 16.5 | 62.4 | 68 |
| | 25.8 | 40.0 | 100 |
| | 30.4 | 34.1 | 87 |
| Dihydrate | 9.2 | 111.6 | 83 |
| | 18.5 | 56.0 | 100 |
| | 34.3 | 30.4 | 77 |

The X-ray data confirm the existence of the three different crystalline forms of aligerone.

Utilisation together of infrared spectra, X-ray data and water content determined according to the method of K. Fischer provides a reliable means of establishing and identifying each of the three crystalline modifications when present, either in the pure state and in mixtures. Additional characterisation may be achieved by using data from differential calorimetry and differential gravitometry.

For this additional characterisation, samples weighing about 100 mg were submitted to analysis in platinum crucibles in an atmosphere of air, utilising a heating rate of 5°C/min. Measurements obtained were recorded by a derivatograph MOM, Budapest. The differential calorimetric data for forms "A" and "B" shows an identical behaviour which differs from that of the crystalline hydrate. Thermogravitometry proves that crystalline modifications "A" and "B" undergo decomposition prior to reaching melting temperature. The decomposition process starts at 180°C and by the time 350°C is reached, the loss in weight amounts to 88.5% of the initial weight of the sample. This makes it difficult to interpret the differential calorimetric data obtained or to determine the melting point with great accuracy.

The crystalline hydrate undergoes two stages of weight loss when subjected to similar heating: (a) a first period from 50°C to 115°C when as a result of dehydration, there is a loss of about 10% of the initial weight (the theoretical water content is 8.98% while analysis according to the method of K. Fischer shows it to be 9.5%). (b) A stage from 160° to 350°C when a loss of 88% of the initial weight takes place as a result of decomposition of the anhydrous substance.

Hygroscopicity studies have been carried out by placing samples of the three crystalline forms with the same particle size resulting from grinding of samples of the three crystalline forms in a vibratory mill. The studies take place in a chamber with 90% humidity and at a temperature of 25°C. At intervals, samples are taken in order to determine their water content and their crystalline structure by means of the methods described hereinabove. The results show that all three forms do not absorb water over a period of 30 days. They are not hygroscopic and are physically stable under the test conditions.

More exhaustive stability studies have been performed under ambient storage conditions in glass jars which permit the passage of light therethrough. Studies at intervals over one year and after three years show that no visual and chemical changes occurred. The three crystalline forms are chemically and physically stable under normal storage conditions.

Table 3 indicates comparative data concerning the stability of injectible forms of pharmaceutical compositions containing either crystalline form "A" according to the present invention and form "B" (aligerone prepared according to the method described in the aforementioned references to Zikolova and Ninov).

TABLE 3
Stability in aqueous solution of different crystalline forms of aligerone

| Crystalline form | Content of active substance in the solution in % | | |
|---|---|---|---|
| | | In storing | |
| | Initial concentration | 1 year | 3 years |
| A | 1.251 | 1.250 | 1.198 |
| B | 1.255 | 1.105 | 0.925 |

The respective solubilities of the three crystalline modifications have also been studied. The rate of dissolution of pulverous specimens in water was determined in the following manner: a finely ground powder formed from the crystalline form under study in a quantity twice as great as necessary to produce a saturated solution in water is placed in a receptacle containing 100 ml of distilled water. The suspension is stirred using a magnetic agitator (600 r.p.m.) at a temperature of 25°C. At predetermined time intervals, samples are taken and, after filtering through a Milipor filter (0.45 µm) they are diluted to the necessary volume for performing a quantitative determination. For this purpose, an absorption maximum at 250 nm is used.

Figure 5 of the accompanying drawings shows the solubility profiles of the powder specimens of the three crystalline forms. It is clear that the anhydrous form "A" attains maximum solubility within 2 minutes and afterwards its solubility decreases until it reaches a constant value after 10 minutes. The solubility of the crystalline hydrate form and of form "B" increases gradually and reaches a constant value only after 60 minutes. A comparison of the solubility profiles of the three crystalline forms shows that the anhydrous form "A" has much higher solubility within the early stages of dissolution as well as a higher equilibrium solubility in comparison with anhydrous form "B" and the crystalline hydrate. This is an important advantage of form "A" which makes it a preferred form for use in the preparation of sterile injectable solutions as well as solid pharmaceutical preparations.

In Table 4 which follows, there are compared the rates of dissolution of the three crystalline forms. Investigations were carried out with pressed discs made up of 200 mg of the test substance and 100 mg of polyvinylchloride pressed at a pressue of 10 tonnes for 5 minutes in a plane around the disc using a pill making matrix with a diameter of 13 mm. The discs thus obtained are immersed by means of a special holding device in 100 ml of 0.1 N hydrochloric acid solution. Stirring was carried out by means of an electromagnetic agitator. The testing temperature employed was 25°C.

TABLE 4

| Crystal form type | Initial rate of dissolving $mg \cdot ml^{-1} \cdot min$ |
|---|---|
| A | 1,380 |
| B | 0.467 |
| Crystalline hydrate | 0.333 |

The initial rates of dissolution are determined from the dissolution curves for the pressed discs, using the linear part between the first and fifth minutes. Comparison of data for the initial rate of dissolution of the three crystalline forms indicate that crystal form "A" has an initial rate of dissolution which is 3 to 4 times greater than that of the other forms.

Thus, it can be seen that investigations which have been carried out show that form "A" is crystalline, anhydrous, non-hygroscopic and stable on storage under normal conditions, including exposure to sunlight. This form of aligerone is prepared easily in reproducible manner.

The crystalline hydrate and anhydrous form "B" are not readily prepared in the pure state and in general they are formed in admixture with one another.

A very important advantage of the anhydrous form "A" is its better solubility in water and in 0.1 N.HCl. Thus, form "A" will be eminently suitable for dissolution in water to make up sterile injectible solutions and also for taking orally for resorption in the gastrointestinal system. Hence anhydrous crystalline form "A" is the preferred form for preparing ready-to-use pharmaceutical compositions.

Form "A" of aligerone may be utilised by itself or in combination with pharmacologically acceptable carriers. The proportion of the active component will depend upon the selected manner in which it is to be used. It can be utilised in the form of pills, capsules or injectible solutions.

The following Examples illustrate the invention:

Example 1

Preparation of 1-benzhydryl-4-allyl-piperazine dihydrochloride in crystalline form "A"

7.8 g of allyl chloride were added dropwise to a solution of 25.2 g of 1-benzhydrylpiperazine and 4 g of sodium hydroxide in a mixture of 65 ml of ethanol and 6.5 ml of water. Stirring was carried out at this temperature for 4 hours. After cooling to 10°C, acidifying with 25 ml of concentrated hydrochloric acid and drying the crystals which had precipitated, 24.5 g of 1-benzhydryl-4-allyl-piperazine dihydrochloride of form "A" were obtained.

Elemental analysis: %C = 65.51;
%H = 7.03;
%N = 7.48;
%Cl = 19.39.

IR-spectrum: $A_{646}/A_{665}$ = 0.6 and $A_{926}/A_{952}$ = 0.8.

The X-ray pattern of a powder specimen of the form "A" showed the most intensive diffraction band at 27.4 (2θ) corresponding to 37.8 nm.

Example 2

Preparation of 1-benzhydryl-4-allyl-piperazine dihydrochloride of crystalline form "A"

25.2 g of 1-benzhydrylpiperazine were reacted with 7.8 g of allyl chloride in a reaction medium made by mixing 90 ml of methanol and 8 ml of water. The reaction was carried out in the presence of 4 g of sodium hydroxide at a temperature of 30°C over a period of 4 hours. The reaction mixture then obtained was cooled, acidified with 25 ml of concentrated hydrochloric acid and the crystalline precipitate formed was filtered off. 19.5 g of 1-benzhydryl-4-allyl piperazine dihydrochloride of crystalline form "A" were obtained.

Elemental analysis: %C = 65.56;
%H = 7.10;
%N = 7.52;
%Cl = 19.35.

IR spectrum: $A_{646}/A_{665}$ = 0.6 and $A_{926}/A_{952}$ = 0.8.

The X-ray diffraction pattern of a powder specimen showed most intensive diffraction bands at 27.4 (2θ), corresponding to 37.8 nm.

Example 3

Preparation of 1-benzhydryl-4-allyl piperazine dihydrochloride (form "A") from 1-benzhydryl-4-allylpiperazine dihydrochloride dihydrate (crystalline hydrate form)

20 g of 1-benzhydryl-4-allylpiperazine dihydrochloride dihydrate were dissolved in a mixture of ethanol-water in a ratio by volume of 9:1 and filtration was carried out while warm to separate off undissolved matter. After cooling, filtering again and drying, a residue of 15.5 g of 1-benzhydryl-4-allyl-piperazine dihydrochloride of form "A" was obtained.

Elemental analysis: %C = 65.63;
%H = 7.11;
%N = 7.56;
%Cl = 19.31.

IR spectrum: $A_{646}/A_{665}$ = 0.6 and $A_{926}/A_{952}$ = 0.8.

The X-ray diffraction pattern of a powder specimen showed the most intensive diffraction band at 27.4 (2θ) which corresponds to 37.8 nm.

Example 4

Preparation of 1-benzhydryl-4-allyl-piperazine dihydrochloride of form "A" from 1-benzhydryl-4-allyl-piperazine dihydrochloride of form "B"

20 g of 1-benzhydryl-4-allylpiperazine dihydrochloride of form "B" were dissolved in 100 ml of a mixture of methanol and water in a volume ratio of 10:1 and filtration of the solution thereby obtained was carried out while the solution was still warm. After cooling the filtrate and filtering off the then suspended matter and drying it, 16.5 g of 1-benzhydryl-4-allylpiperazine dihydrochloride of form "A" were obtained.

Elemental analysis: %C = 65.48;
%H = 7.09;
%N = 7.55;
%Cl = 19.40.

IR spectrum: $A_{646}/A_{665}$ = 0.6 and $A_{926}/A_{952}$ = 0.8.

The X-ray diffraction pattern of a powder specimen showed the most intensive diffraction band at 27.4 (2θ), corresponding to 37.8 nm.

**Claims for the Contracting States: BE FR DE IT SE CH GB**

1. An anhydrous crystalline form of 1-benzhydryl-4-allylpiperazine dihydrochloride which possesses absorption bands in its IR-spectra (tablets with nujol mulls) at 646 and 665 $cm^{-1}$, a ratio $A_{646}/A_{661} = 0.6$, a specific triplet at 915, 926 and 952 $cm^{-1}$, a ratio $A_{926}/A_{952} = 0.8$, absence of a band at 3380 $cm^{-1}$ and a strongest X-ray diffraction band at 27.4 (2θ) corresponding to 37.8 nm.

2. A method for the preparation of the anhydrous crystalline form of 1-benzhydryl-4-allylpiperazine dihydrochloride of claim 1, which comprises providing in solution in an aqueous alcoholic medium either

(a) 1-benzhydryl-4-allylpiperazine, as such and then acidifying this solution with concentrated hydrochloric acid, forming therein a crystalline precipitate of 1-benzhydryl-4-allylpiperazine dihydrochloride and separating off the crystalline precipitate from the liquor in which it has been formed, or

(b) 1-benzhydryl-4-allylpiperazine dihydrochloride in a crystalline form other than its anhydrous crystalline form, forming therein a crystalline precipitate of 1-benzhydryl-4-allylpiperazine dihydrochloride and then separating off the crystalline precipitate from the liquor in which it has been formed.

3. A method as claimed in claim 2(a), wherein the 1-benzhydryl-4-allylpiperazine as such has been prepared *in situ* in the aqueous alcoholic medium by reacting 1-benzhydrylpiperazine therein with an allyl halide in the presence of an alkaline condensation agent, reaction taking place at a temperature of from 0 to 40°C.

4. A method as claimed in claim 2(a) or 3, wherein the alcohol is methanol, ethanol or propanol.

5. A method as claimed in claim 4, wherein the aqueous alkaline medium is ethanol containing from 5 to 30% by volume of water.

6. A method as claimed in any one of claims 3 to 5, wherein reaction between the 1-benzhydrylpiperazine and allyl halide takes place at about 25°C.

7. A pharmaceutical composition which comprises 1-benzhydryl-4-allylpiperazine dihydrochloride as claimed in claim 1 in association with a pharmacologically acceptable adjuvant.

8. A pharmaceutical composition as claimed in claim 7, which is in the form of a sterile injectable solution.

**Claims for the Contracting State: AT**

1. A method for the preparation of an anhydrous crystalline form of 1-benzhydryl-4-allylpiperazine dihydrochloride, which possesses absorption bands in its IR-spectra (tablets with nujol mulls) at 646 and 665 $cm^{-1}$, a ratio $A_{646}/A_{661} = 0.6$, a specific triplet at 915, 926 and 952 $cm^{-1}$, a ratio $A_{926}/A_{952} = 0.8$, absence of a band at 3380 $cm^{-1}$ and a strongest X-ray diffraction band at 27.4 (2θ) corresponding to 37.8 nm which comprises providing in solution in an aqueous alcoholic medium either

(a) 1-benzhydryl-4-allylpiperazine, as such and then acidifying this solution with concentrated hydrochloric acid, forming therein a crystalline precipitate of dihydrochloride and separating off the crystalline precipitate from the liquor in which it has been formed, or

(b) 1-benzhydryl-4-allylpiperazine dihydrochloride in a crystalline form other than its anhydrous crystalline form, forming therein a crystalline precipitate of 1-benzhydryl-4-allylpiperazine dihydrochloride and then separating off the crystalline precipitate from the liquor in which it has been formed.

2. A method as claimed in claim 1(a), wherein the 1-benzhydryl-4-allylpiperazine has been prepared *in situ* in the aqueous alcoholic medium by reacting 1-benzhydrylpiperazine therein with an allyl halide in the presence of an alkaline condensation agent, reaction taking place at a temperature of from 0 to 40°C.

3. A method as claimed in claim 1(a) or 2, wherein the alcohol is methanol, ethanol or propanol.

4. A method as claimed in claim 3, wherein the aqueous alkaline medium is ethanol containing from 5 to 30% by volume of water.

5. A method as claimed in any one of claims 2 to 4, wherein reaction between the 1-benzhydrylpiperazine and allyl halide takes place at about 25°C.

6. A method for the production of a pharmaceutical composition, which comprises compounding an anhydrous crystalline form of 1-benzhydryl-4-allylpiperazine dihydrochloride, which possesses absorption bands in its IR-spectra (tablets with nujol mulls) at 646 and 665 $cm^{-1}$, a ratio $A_{646}/A_{661} = 0.6$, a specific triplet at 915, 926 and 952 $cm^{-1}$, a ratio $A_{926}/A_{952} = 0.8$, absence of a band at 3380 $cm^{-1}$ and a strongest X-ray diffraction band at 27.4 (2θ) corresponding to 37.8 nm, with a pharmacologically acceptable adjuvant.

7. A method as claimed in claim 6, wherein a sterile injectable solution is produced by dissolving said anhydrous crystalline form of 1-benzhydryl-4-allylpiperazine dihydrochloride in water.

**Patentansprüche für die Vertragsstaaten: BE FR DE IT SE CH GB**

1. Wasserfreie kristalline Form von 1-Benzhydryl-4-allylpiperazin-dihydrochlorid, das Absorptionsbanden in seinen IR-Spektra (Tabletten mit Nujolmull) bei 646 und 665 $cm^{-1}$, ein Verhältnis $A_{646}/A_{661} = 0,6$, ein spezifisches Triplett bei 915, 926 und 952 $cm^{-1}$, ein Verhältnis $A_{926}/A_{952} = 0,8$, Abwesenheit einer Bande bei 3380 $cm^{-1}$ und eine stärkste Röntgenbeugungsbande bei 27,4 2, was 37,8 nm entspricht, zeigt.

2. Verfahren zur Herstellung der wasserfreien kristallinen Form von 1-Benzhydryl-4-allylpiperazin-dihydrochlorid nach Anspruch 1, bei dem man in einem wässerigen alkoholischen Medium entweder

a) 1-Benzhydryl-4-allylpiperazin als solches in Lösung bringt und diese Lösung dann mit konz. Salzsäure ansäuert, wodurch ein kristallines Präzipitat von 1-Benzhydryl-4-allylpiperazin-dihydrochlorid gebildet wird, wonach das kristalline Präzipitat aus der Flüssigkeit, in der es gebildet wurde, abgetrennt wird oder

b) 1-Benzhydryl-4-allylpiperazin-dihydrochlorid in einer von der wasserfreien kristallinen Form unterschiedlichen kristallinen Form in Lösung gebracht wird, wodurch ein kristallines Präzipitat von 1-Benzhydryl-4-allylpiperazin-dihydrochlorid gebildet wird, wonach das kristalline Präzipitat aus der Flüssigkeit, in der es gebildet wurde, abgetrennt wird.

3. Verfahren nach Anspruch 2a), wobei das 1-Benzhydryl-4-allylpiperazin als solches in situ in dem wässerigen alkoholischen Medium durch Umsetzung von 1-Benzhydrylpiperazin mit einem Allylhalogenid in Anwesenheit eines alkalischen Kondensationsmittels hergestellt wurde, wobei die Reaktion bei einer Temperatur von 0 bis 40°C durchgeführt wird.

4. Verfahren nach Anspruch 2a), oder 3, wobei der Alkohol Methanol, Ethanol oder Propanol ist.

5. Verfahren nach Anspruch 4, wobei das wässerige alkalische Medium Ethanol ist, der 5 bis 30 Vol.-% Wasser enthält.

6. Verfahren nach einem der Ansprüche 3 bis 5, wobei die Reaktion zwischen 1-Benzhydryl-piperazin und Allylhalogenid bei ca. 25°C durchgeführt wird.

7. Pharmazeutische Zusammensetzung, die 1-Benzhydryl-4-allylpiperazin-dihydrochlorid nach Anspruch 1 in Verbindung mit einem pharmakologisch verträglichen Zusatz enthält.

8. Pharmazeutische Zusammensetzung nach Anspruch 7, die in Form einer sterilen injizierbaren Lösung vorliegt.

**Patentansprüche für den Vertragsstaat: AT**

1. Verfahren zur Herstellung einer wasserfreien kristallinen Form von 1-Benzhydryl-4-allylpiperazin-dihydrochlorid, das Absorptionsbanden in seinen IR-Spektra (Tabletten mit Nujolmull) bei 646 und 665 $cm^{-1}$, ein Verhältnis $A_{646}/A_{661}$ = 0,6, ein spezifisches Triplett bei 915, 926 und 952 $cm^{-1}$, ein Verhältnis $A_{926}/A_{952}$ = 0,8, Abwesenheit einer Bande bei 3380 $cm^{-1}$ und eine stärkste Röntgenbeugungsbande bei 27,4 2, was 37,8 nm entspricht, zeigt bei dem man in einem wässerigen alkoholischen Medium entweder

a) 1-Benzhydryl-4-allylpiperazin als solches in Lösung bringt und diese Lösung dann mit konz. Salzsäure ansäuert, wodurch ein kristallines Präzipitat von 1-Benzhydryl-4-allylpiperazin-dihydrochlorid gebildet wird, wonach das kristalline Präzipitat aus der Flüssigkeit, in der es gebildet wurde, abgetrennt wird oder

b) 1-Benzhydryl-4-allylpiperazin-dihydrochlorid in einer von der wasserfreien kristallinen Form unterschiedlichen kristallinen Form in Lösung gebracht wird, wodurch ein kristallines Präzipitat von 1-Benzhydryl-4-allylpiperazin-dihydrochlorid gebildet wird, wonach das kristalline Präzipitat aus der Flüssigkeit, in der es gebildet wurde, abgretrennt wird.

2. Verfahren nach Anspruch 1a), wobei das 1-Benzhydryl-4-allylpiperazin als solches in situ in dem wässerigen alkoholischen Medium durch Umsetzung von 1-Benzhydrylpiperazin mit einem Allylhalogenid in Anwesenheit eines alkalischen Kondensationsmittels hergestellt wurde, wobei die Reaktion bei einer Temperatur von 0 bis 40°C durchgeführt wird.

3. Verfahren nach Anspruch 1a), oder 2, wobei der Alkohol Methanol, Ethanol oder Propanol ist.

4. Verfahren nach Anspruch 3, wobei das wässerige alkalische Medium Ethanol ist, der 5 bis 30 Vol.-% Wasser enthält.

5. Verfahren nach einem der Ansprüche 2 bis 4, wobei die Reaktion zwischen 1-Benzhydryl-piperazin und Allylhalogenid bei ca. 25°C durchgeführt wird.

6. Verfahren zur Herstellung einer pharmazeutischen Zusammensetzung, welches das Vermischen einer wasserfreien kristallinen Form von 1-Benzhydryl-4-allylpiperazin-dihydrochlorid, das Absorptionsbanden in seinen IR-Spektra (Tabletten mit Nujolmull) bei 646 und 665 $cm^{-1}$, ein Verhältnis $A_{646}/A_{661}$ = 0,6 ein spezifisches Triplett bei 915, 926 und 952 $cm^{-1}$, ein Verhältnis $A_{926}/A_{952}$ = 0,8, Abwesenheit einer Bande bei 3380 $cm^{-1}$ und eine stärkste Röntgenbeugungsbande bei 27,4 2, was 37,8 nm entspricht, zeigt mit einem pharmakologisch verträglichen Zusatz umfaßt.

7. Verfahren nach Anspruch 6, wobei die sterile injizierbare Lösung durch Lösen der wasserfreien kristallinen Form von 1-Benzhydryl-4-allylpiperazin-dihydrochlorid, in Wasser hergestellt wird.

**Revendications pour les Etats Contractants: BE FR DE IT SE CH GB**

1. Forme cristalline anhydre du dichlorhydrate de benzhydryl-1 allyl-4 pipérazine qui possède des bandes d'absorption dans ses spectres IR (pastilles de pâte au Nujol) à 646 et 665 $cm^{-1}$, un rapport $A_{646}/A_{661}$ = 0,6, un triplet spécifique à 915, 926 et 952 $cm^{-1}$, un rapport $A_{926}/A_{952}$ = 0,8, une absence de bande à 3380 $cm^{-1}$ et une bande de diffraction des rayons X la plus forte à 27,4, 2θ, correspondant à 37,8 nm.

2. Procédé de préparation de la forme cristalline anhydre du dichlorhydrate de benzhydryl-1 allyl-4 pipérazine de la revendication 1, qui comprend les étapes consistant à amener en solution dans un milieu

alcoolique aqueux

(a) ou bien de la benzhydryl-1 allyl-4 pipérazine en tant que telle, puis à acidifier cette solution par de l'acide chlorhydrique concentré, formant ainsi en son sein un précipité cristallin de dichlorhydrate de benzhydryl-1 allyl-4 pipérazine, et à séparer le précipité cristallin de la liqueur dans laquelle il a été formé,

(b) ou bien du dichlorhydrate de benzhydryl-1 allyl-4 pipérazine sous une forme cristalline autre que sa forme cristalline anhydre, formant ainsi en son sein un précipité cristalline de dichlorhydrate de benzhydryl-1 allyl-4 pipérazine, puis à séparer le précipité cristallin de la liqueur dans laquelle il a été formé.

3. Procédé selon la revendication 2(a), dans lequel on a préparé la benzhydryl-1 allyl-4 pipérazine en tant que telle *in situ* dans le milieu alcoolique aqueux, en faisant réagir en son sein de la benzhydryl-1 pipérazine avec un halogénure d'allyle, en présence d'un agent de condensation alcalin, la réaction ayant lieu à une température de 0 à 40°C.

4. Procédé selon la revendication 2(a) ou 3, dans lequel l'alcool est le méthanol, l'éthanol ou le propanol.

5. Procédé selon la revendication 4, dans lequel le milieu alcalin aqueux est de l'éthanol contenant de 5 à 30% en volume d'eau.

6. Procédé selon l'une des revendications 3 à 5, dans lequel la réaction entre la benzhydryl-1 pipérazine et l'halogénure d'allyle a lieu à environ 25°C.

7. Composition pharmaceutique qui comprend du dichlorhydrate de benzhydryl-1 allyl-4 pipérazine tel que défini à la revendication 1, en association avec un adjuvant pharmacologiquement acceptable.

8. Composition pharmaceutique selon la revendication 7, qui se présente sous la forme d'une solution injectable stérile.

**Revendications pour l'Etat Contractant: AT**

1. Procédé de préparation d'une forme cristalline anhydre du dichlorhydrate de benzhydryl-1 allyl-4 pipérazine qui possède des bandes d'absorption dans ses spectres IR (pastilles de pâte au Nujol) à 646 et 665 $cm^{-1}$, un rapport $A_{646}/A_{661}$ = 0,6, un triplet spécifique à 915, 926 et 952 $cm^{-1}$, un rapport $A_{926}/A_{952}$ = 0,8, une absence de bande à 3380 $cm^{-1}$ et une bande de diffraction des rayons X la plus forte à 27,4, 2θ, correspondant à 37,8 nm, qui comprend les étapes consistant à amener en solution dans un milieu alcoolique aqueux

(a) ou bien de la benzhydryl-1 allyl-4 pipérazine en tant que telle, puis à acidifier cette solution par de l'acide chlorhydrique concentré, formant ainsi en son sein un précipité cristallin de dichlorhydrate, et à séparer le précipité cristallin de la liqueur dans laquelle il a été formé,

(b) ou bien du dichlorhydrate de benzhydryl-1 allyl-4 pipérazine sous une forme cristalline autre que sa forme cristalline anhydre, formant ainsi en son sein un précipité cristallin de dichlorhydrate de benzhydryl-1 allyl-4 pipérazine, puis à séparer le précipité cristallin de la liqueur dans laquelle il a été formé.

2. Procédé selon la revendication 1(a), dans lequel on a préparé la benzhydryl-1 allyl-4 pipérazine *in situ* dans le milieu alcoolique aqueux, en faisant réagir en son sein de la benzhydryl-1 pipérazine avec un halogénure d'allyle, en présence d'un agent de condensation alcalin, la réaction ayant lieu à une température de 0 à 40°C.

3. Procédé selon la revendication 1(a) ou 2, dans lequel l'alcool est le méthanol, l'éthanol ou le propanol.

4. Procédé selon la revendication 3, dans lequel le milieu alcalin aqueux est de l'éthanol contenant de 5 à 30% en volume d'eau.

5. Procédé selon l'une des revendications 2 à 4, dans lequel la réaction entre la benzhydryl-1 pipérazine et l'halogénure d'allyle a lieu à environ 25°C.

6. Procédé de fabrication d'une composition pharmaceutique, qui comprend la formulation d'une forme cristalline anhydre du dichlorhydrate de benzhydryl-1 allyl-4 pipérazine, qui possède des bandes d'absorption dans ses spectres IR (pastilles de pâte au Nujol) à 646 et 665 $cm^{-1}$, un rapport $A_{646}/A_{661}$ = 0,6, un triplet spécifique à 915, 926 et 952 $cm^{-1}$, un rapport $A_{926}/A_{952}$ = 0,8, une absence de bande à 3380 $cm^{-1}$ et une bande de diffraction des rayons X la plus forte à 27,4, 2θ, correspondant à 37,8 nm, avec un adjuvant pharmacologiquement acceptable.

7. Procédé selon la revendication 6, dans lequel on produit une solution injectable stérile en dissolvant dans l'eau ladite forme cristalline anhydre du dichlorhydrate de benzhydryl-1 allyl-4 pipérazine.

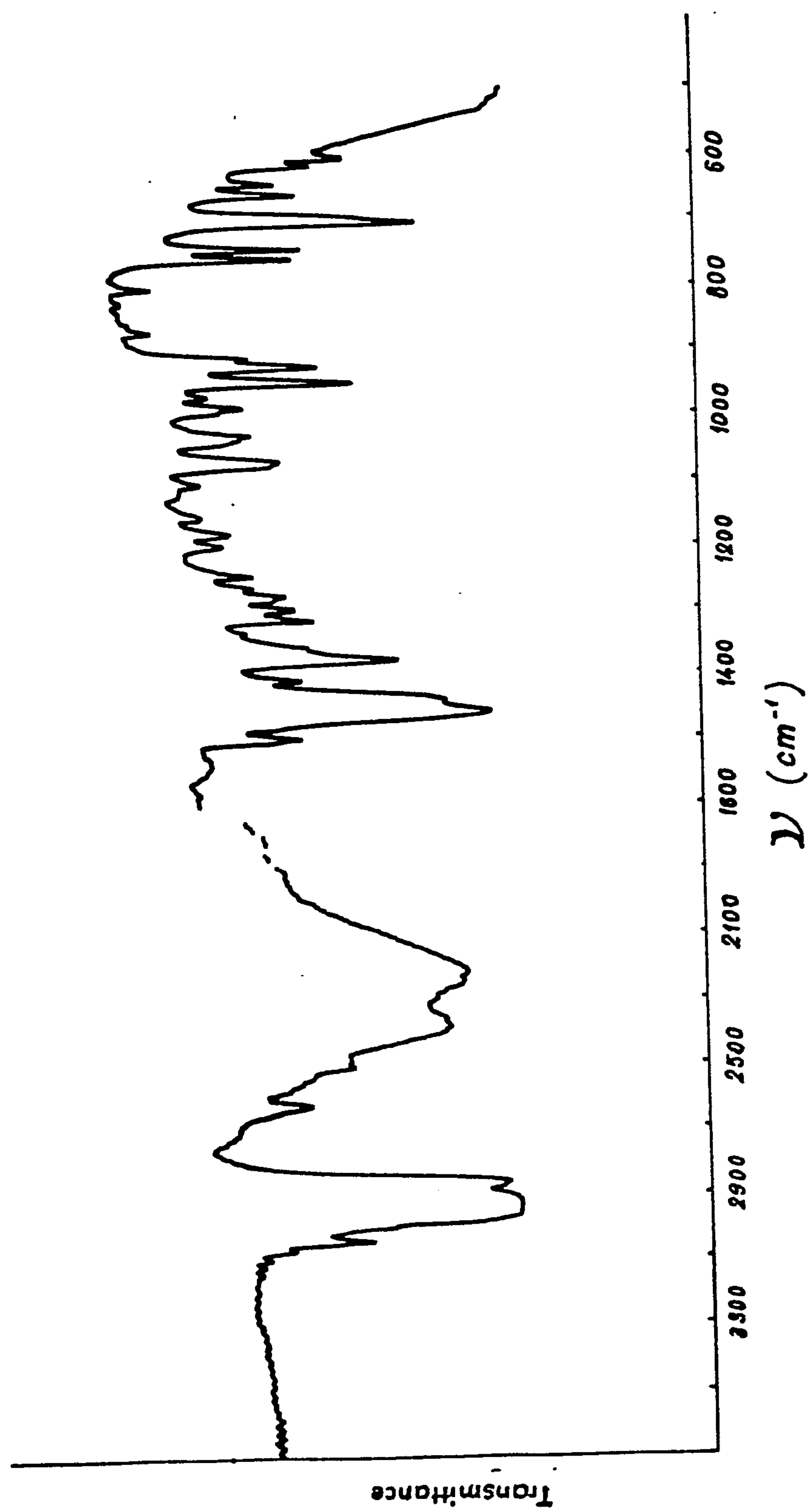
Transmittance
600
800
1000
1200
1400
1600
2100
2500
2900
3300
ν (cm-1)

Fig. 1

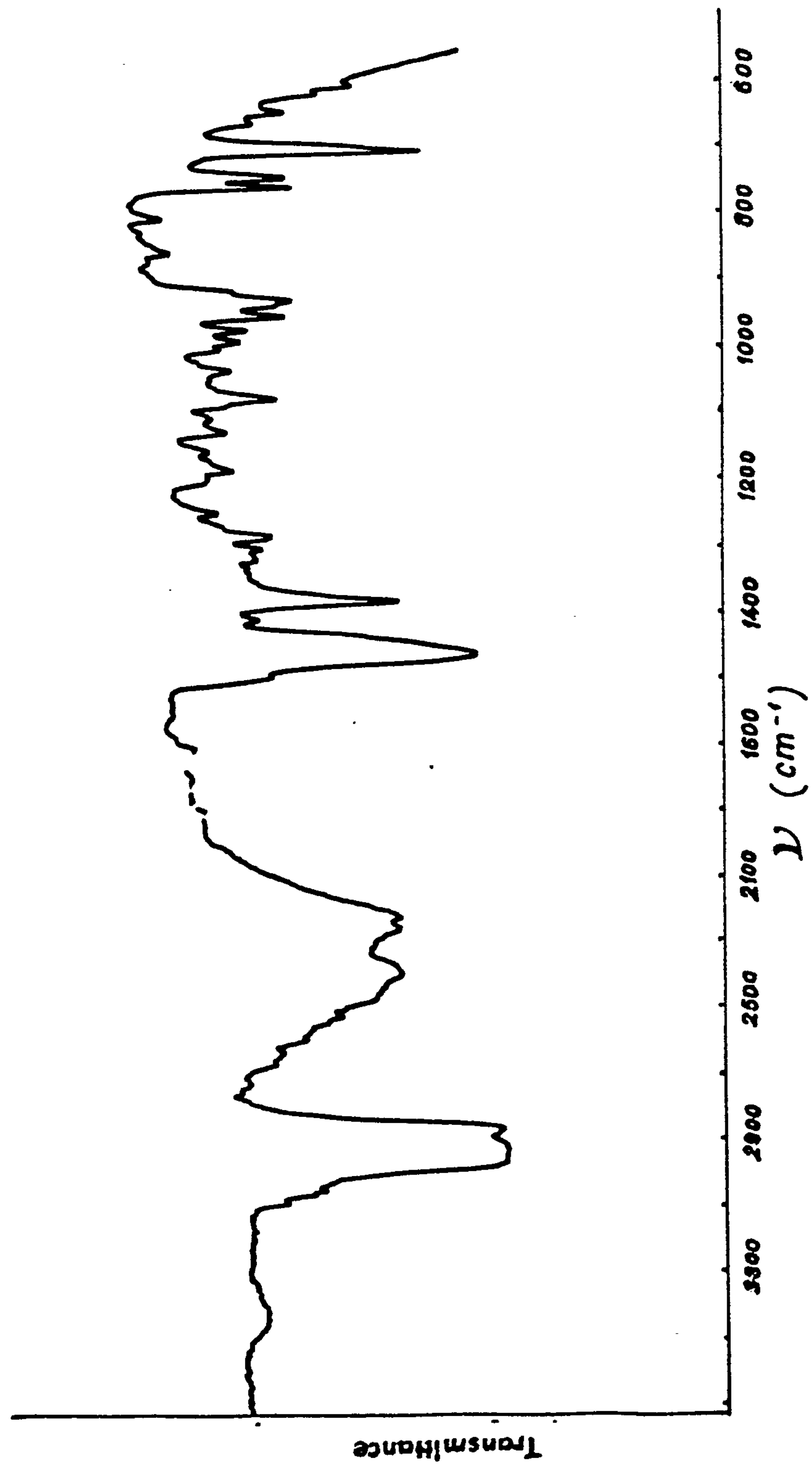
600
800
1000
1200
1400
1600
2100
2500
2900
3300
ν (cm⁻¹)
Transmittance

Fig. 2

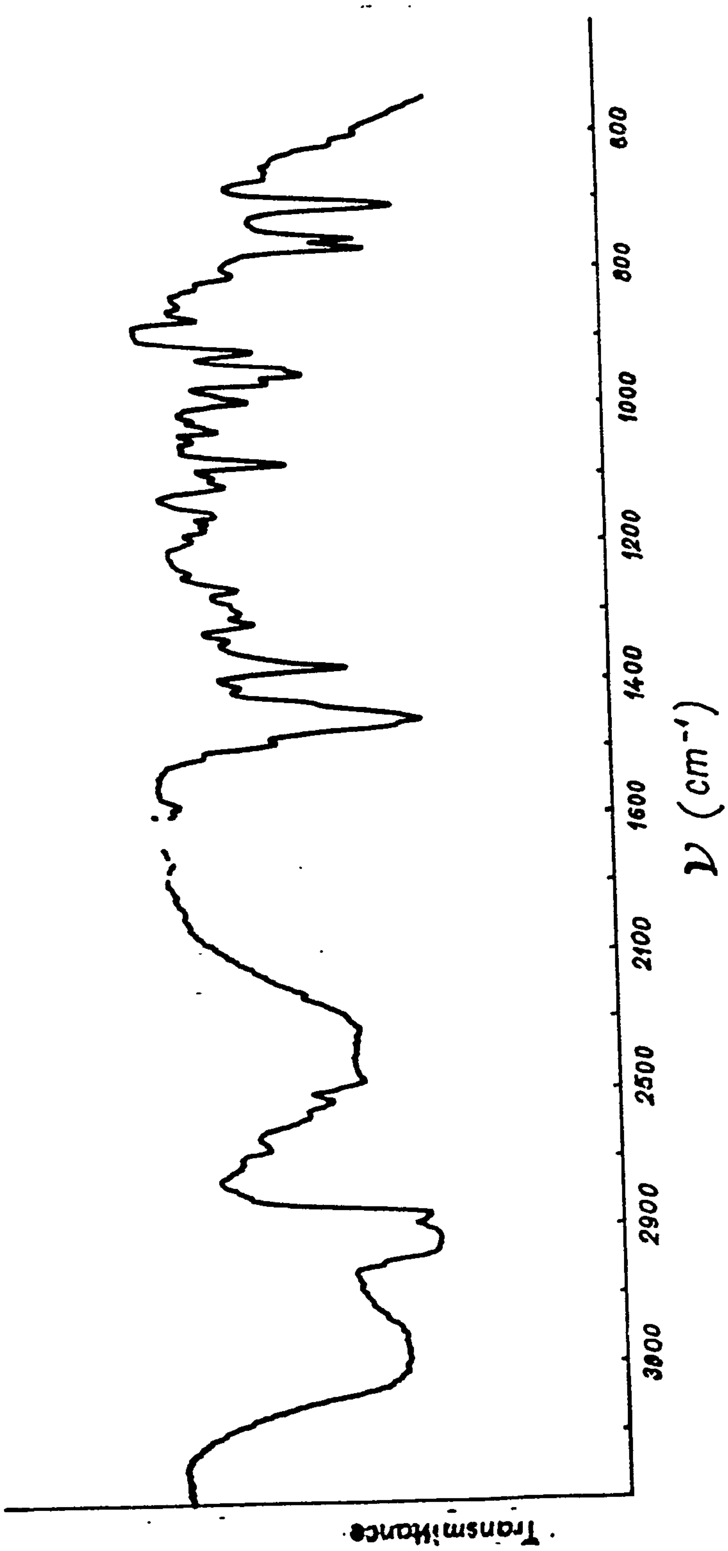
600
800
1000
1200
1400
1600
2100
2500
2900
3300
ν (cm⁻¹)
Transmittance

Fig. 3

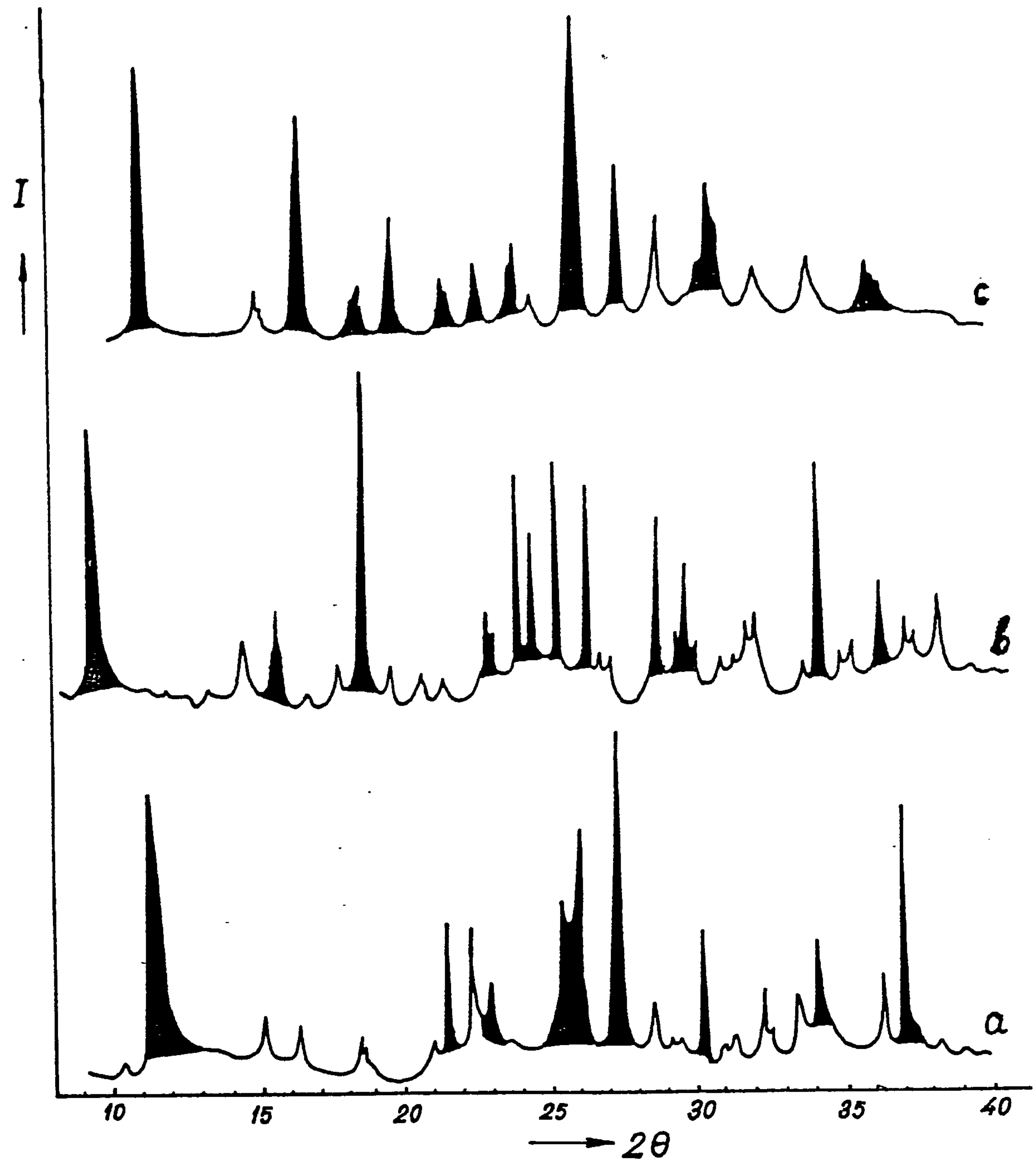


Fig.4

X-ray diffraction patterns of :

a/Form A , b/ Dihydrate , c/ Form B

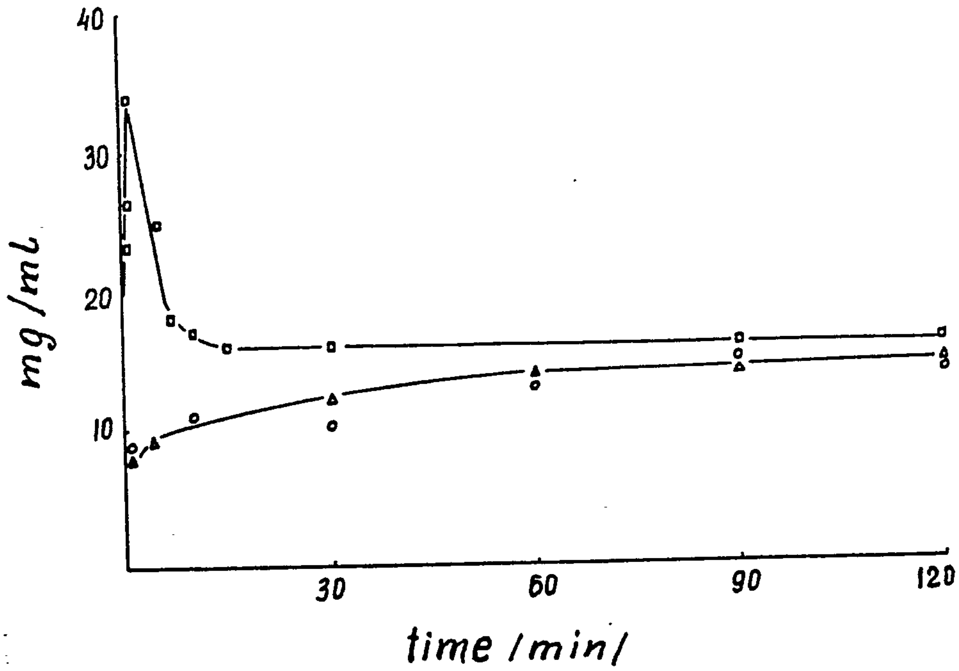


Fig. 5

Dissolution profiles of :

-□-□-□- Form A

-o-o-o- Form B

-Δ-Δ-Δ- Form Dihydrate